# EUROPÄISCHE PATENTSCHRIFT

(11) **EP 0 847 994 B1**
(45) Veröffentlichungstag und Bekanntmachung des Hinweises auf die Patenterteilung: **09.04.2003**
(21) Anmeldenummer: 97121298.0
(22) Anmeldetag: 04.12.1997
(51) Int. Cl.: C07D 215/48, C07D 211/46, C07C 271/22, C07C 51/56

(54) **Verfahren zur Herstellung gemischter Anhydride**
Process for the preparation of mixed anhydrides
Procédé pour la préparation d'anhydrides mixtes

(30) Priorität: 11.12.1996 EP 96119853
(43) Veröffentlichungstag der Anmeldung: 17.06.1998
(73) Patentinhaber: F. HOFFMANN-LA ROCHE AG, 4070 Basel (CH)
(72) Erfinder: Karpf, Martin, 4153 Reinach (CH); Trussardi, René, 4127 Birsfelden (CH)
(74) Vertreter: Witte, Hubert, Dr.

(56) Entgegenhaltungen:
- US-A- 4 874 558
- CHEMICAL ABSTRACTS, vol. 108, no. 21, 23.Mai 1988 Columbus, Ohio, US; abstract no. 186589a, MASAKI,MITSUO ET AL: "Novel quinaldinamide derivatives, ..." XP002060743 & ZA 8 606 196 A (NIPPON CHEMIPHAR CO.,LTD.)
- CHEMICAL ABSTRACTS, vol. 120, no. 25, 20.Juni 1994 Columbus, Ohio, US; abstract no. 323378v, REITZ,DAVID B. ET AL: "N1-sterically hindered 2-Himidazol-2-one ..." XP002060744 & BIOORG. MED. CHEM. LETT. , Bd. 4, Nr. 1, - 1994 Seiten 111-114,
- CHEMICAL ABSTRACTS, vol. 105, no. 1, 7.Juli 1986 Columbus, Ohio, US; abstract no. 6541f, LAPSHIN,S,H, ET AL: "Stable N-acylimidazole salts." XP002060745 & UKR. KHIM. ZH., Bd. 51, Nr. 8, - 1985 Seiten 860-865,
- Justus Liebigs Ann. Chem. 1937, 530, 34 - 45
- Helv. Chim. Acta, 1994, 77, 575 - 578
- Tetrahedron, 1984, 40, 4415 - 4424

## Beschreibung

Die vorliegende Erfindung betrifft ein verbessertes Verfahren zur Herstellung gemischter Anhydride.

Die Herstellung gemischter Anhydride ist an sich bekannt und beruht auf der Umsetzung einer Säure mit einem reaktiven Säurederivat, z. B. einem Säurehalogenid oder Säureanhydrid unter Anwesenheit einer Hilfsbase. Die Herstellung gemischter Anhydride ist zum Beispiel von Bodansky in "Principles of Peptide Synthesis", 2nd ed., Springer-Verlag, Berlin, 1993, Seiten 21 - 29 und in "The Practice of Peptide Synthesis", 2nd ed., Springer Verlag, Berlin, 1994 sowie von Stelzel in Houben-Weyl, Band XV/2, "Methoden der organischen Chemie: "Synthese von Peptiden", Teil II, beschrieben worden.

Gemischte Anhydride spielen vor allem bei Aktivierungs- und Kupplungs-reaktionen eine Rolle. So beschreibt Bodansky (loc. cit.) die Synthese gemischter Anhydride mit Pivaloylchlorid zur Verwendung in der Peptidsynthese. Beispielsweise werden zur Herstellung von Benzyloxycarbonyl-α-methylalanyl-α-methylalanin-methylester Säure und Hilfsbase vorgelegt und das Säurechlorid zugegeben. Die Mischung wird für 2 Stunden bei -5° C und anschliessend für 1 Stunde bei Raumtemperatur gerührt. Analog verfährt man bei der Herstellung von N^{α}-Benzyloxycarbonyl-N^{ε}-p-toluol-sulfonyl-L-lysylglycin-ethylester.

Stelzel (loc. cit.) beschreibt die Synthese von N-Benzyloxycarbonyl-Lprolyl-L-leucylglycin-ethylester basierend auf der Zugabe von Isovaleroylchlorid zu einer Mischung aus Z-Pro-OH und Triethylamin in Toluol.

Diese und andere bekannte Verfahren zur Herstellung gemischter Anhydride weisen jedoch erhebliche Nachteile auf. So verläuft die Reaktion bei weitem nicht quantitativ. Dies ist in erster Linie auf Nebenproduktbildung, z. B. durch Disproportionierung zu den entsprechenden symmetrischen Anhydriden, zurückzuführen. Folglich muss die Reaktionsmischung einer aufwendigen Aufarbeitung und Reinigung unterzogen werden.

Überraschenderweise wurde nun gefunden, dass die oben genannten Nachteile weitgehend vermieden werden können, wenn bei der Herstellung eines gemischten Anhydrids nicht das reaktive Säurederivat zum Gemisch von Säure und Hilfsbase gegeben wird, sondern Säure und reaktives Säurederivat vorgelegt werden und die Base zu der entsprechenden Lösung oder Suspension gegeben wird.

Folglich umfasst die vorliegende Erfindung ein Verfahren zur Herstellung gemischter Anhydride, dadurch gekennzeichnet, dass eine Hilfsbase zum Gemisch aus Säure und reaktivem Säurederivat gegeben wird, worin die Säure eine Carbonsäure ist, die Hilfsbase ein tertiäres Amin ist und das reaktive Säurederivat ein Säurehalogenid einer Säure R¹-COOH oder ein Chlorameisensäurealkylester ist, worin R¹ Alkyl, Cycloalkyl, Heteroaryl oder Aryl ist.

Als "Säuren" im Rahmen der vorliegenden Erfindung kommen Carbonsäuren in Frage. Dabei kann es sich z. B. um unsubstituierte und substituierte aliphatische, aromatische, aromatisch-aliphatische, heteroaromatische oder heteroaromatisch-aliphatische Carbonsäuren oder geschützte Aminocarbonsäuren, z. B. N-acylierte Aminocarbonsäuren, wie natürliche N-acylierte α-Aminosäuren mit L-Konfiguration oder entsprechende nicht-natürliche mit D-Konfiguration sowie die entsprechenden Racemate der L- und D-Aminosäuren, handeln. Ausserdem können Homologe solcher Aminosäuren verwendet werden, z. B. Aminosäuren, worin die Aminosäureseitenkette um eine oder zwei Methylengruppen verlängert oder verkürzt ist und/oder eine Methylgruppe durch Wasserstoff ersetzt ist. Weiterhin können substituierte aromatische N-acylierte α-Aminosäuren, z. B. substituiertes Phenylalanin oder Phenylglycin, verwendet werden, die einen oder mehrere der folgenden Substituenten - unabhängig voneinander - tragen können: Alkyl, z. B. Methyl, Halogen, eine geschützte Hydroxygruppe, Alkoxy, z. B. Methoxy, Alkanoyloxy, z. B. Acetoxy, eine geschützte Amino- oder Alkylaminogruppe, Alkanoylamino, z. B. Acetylamino oder Pivaloylamino, Alkoxycarbonyl-amino, z. B. t-Butoxycarbonylamino, Arylmethoxycarbonylamino, z.B. Benzyloxy-carbonylamino, 9-Fluorenylmethoxycarbonyl und/oder Nitro. Daneben kommt auch benzanneliertes Phenylalanin oder Phenylglycin, wie α-Naphthylalanin, oder hydriertes Phenylalanin oder Phenylglycin, wie Cyclohexylalanin oder Cyclohexylglycin, eine 5- oder 6-gliedrige cyclische benzannelierte N-acylierte α-Aminosäure, z.B. Indolin-2-carbonsäure oder 1,2,3,4-Tetrahydroisochinolin-3-carbonsäure in Frage. Weiterhin können natürliche oder homologe N-acylierte α-Aminosäuren in der eine Carboxygruppe in der Seitenkette in veresterter oder amidierter Form vorliegt, z. B. als Alkylestergruppe, wie Methoxycarbonyl oder t-Butoxycarbonyl, oder als Carbamoylgruppe, Alkylcarbamoylgruppe, wie Methylcarbamoyl, oder als Dialkylcarbamoylgruppe, wie Dimethylcarbamoyl, und in der die Aminogruppe der Seitenkette in acylierter Form vorliegt, z. B. als Alkanoylaminogruppe, wie Acetylamino oder Pivaloylamino, als Alkoxycarbonylaminogruppe, wie t-Butoxycarbonylamino, verwendet werden. Zusätzlich können Aminosäuren, in der eine Carboxygruppe in der Seitenkette vorliegt, als Arylmethoxycarbonylaminogruppe, wie Benzyloxycarbonylamino, eingesetzt werden. Eine Hydroxygruppe der Seitenkette kann in verätherter oder veresterter Form vorliegen, z. B. als Alkoxygruppe, wie Methoxy, aber auch als Arylalkoxygruppe, wie Benzyloxy, oder als nieder-Alkanoyloxygruppe, wie Acetoxy. Geeignete N-Acylgruppen sind Alkanoyl, wie Acetyl oder Pivaloyl, Alkoxycarbonyl, wie t-Butoxycarbonyl, Arylalkoxycarbonyl, wie Benzyloxycarbonyl.

Beispiele geeigneter unsubstituierter und substituierter aliphatischer, aromatischer und aromatisch-aliphatischer Carbonsäuren, die gegebenenfalls in Form ihrer geschützten Derivate verwendete werden können, sind Propionsäure, Isobuttersäure, (R)- und (S)-Milchsäure sowie die entsprechenden Racemate, 2-Phthalimidoxy-isobuttersäure, Benzoesäure, 3,4-Dihydroxybenzoesäure, Salicylsäure, 1-Naphthoesäure, 2-Naphthoesäure, Phenylessigsäure, p-Hydroxyphenyl-essigsäure, (S)-α-[(t-Butylsulfonyl)methyl]hydrozimtsäure und (S)-α-[[[1-(Morpholinocarbonyl)-1-methylethyl]sulfonyl]methyl]-hydrozimtsäure. Als Beispiele geeigneter heteroaromatischer oder heteroaromatisch-aliphatischer Carbonsäuren können 2-Pyridincarbonsäure, 3-Pyridincarbonsäure, 4-Pyridincarbonsäure, 5-Chlor-2-pyridincarbonsäure, 2-Pyrimidincarbonsäure, 4-Pyrimidincarbonsäure, 2-Chinolincarbonsäure, 3-Chinolincarbonsäure, 2-Pyridylessigsäure, 3-Indolylessigsäure, 3-(3-Indolyl)propionsäure, Isochinolin-1-carbonsäure und (4-Imidazolyl)essigsäure genannt werden.

Beispiele geeigneter, oben beschriebener Aminosäuren, die gegebenenfalls in Form ihrer geschützten Derivate verwendete werden können, sind Glycin, Alanin, Valin, Norvalin, Leucin, Isoleucin, Norleucin, Serin, Homoserin, Threonin, Methionin, Cystein, Prolin, Trans-3- und Trans-4-hydroxyprolin, Phenylalanin, Tyrosin, 4-Nitrophenylalanin, 4-Aminophenylalanin, 4-Chlorphenylalanin, β-Phenylserin, Phenylglycin, α-Naphthylalanin, Cyclohexylalanin, Cyclohexyl-glycin, Tryptophan, Indolin-2-carbonsäure, 1,2,3,4-Tetrahydroisochinolin-3-carbonsäure, Asparaginsäure, Asparagin, Aminomalonsäure, Aminomalonsäuremonoamid, Glutaminsäure, Glutaminsäuremono-tbutylester, Glutamin, N-Dimethylglutamin, Histidin, Arginin, Lysin, N-t-Butoxycarbonyllysin, δ-Hydroxylysin, Ornithin, N-Pivaloylornithin, α,γ-Diaminobuttersäure oder α,β-Diaminopropionsäure und dergleichen. Eingeschlossen sind ebenfalls entsprechende Peptide, die z. B. aus den genannten Aminosäuren bestehen.

Bevorzugte Carbonsäuren sind die Chinolin-2-carbonsäure sowie die geschützten Aminocarbonsäuren der Formel I 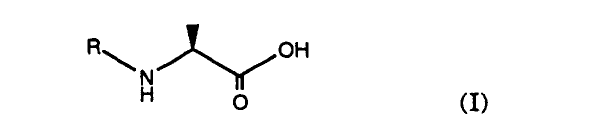 wobei R eine Aminoschutzgruppe ist. Besonders bevorzugte Carbonsäuren sind Chinolincarbonsäuren sowie Säuren der Formel 1, in denen R die Benzyoxycarbonyl- oder die tert.Butoxycarbonyl-Schutzgruppe darstellt, zum Beispiel (S)-2-Benzyloxycarbonylamino-propionsäure oder (S)-2-tert-Butyloxycarbonyl-aminopropionsäure.

Die beschriebenen Säuren sind kommerziell erhältlich bzw. lassen sich durch Umsetzung mit reaktiven Derivaten der Aminoschutzgruppen herstellen.

Der Ausdruck "Alkyl" bezeichnet zyklische, verzweigte oder geradkettige Alkylgruppen mit 1 - 8, bevorzugt 1 - 4 Kohlenstoffatomen.

Der Ausdruck "Aryl" bzw. "Aromat" allein oder in Kombination bezieht sich auf die Phenyl- oder die Naphthylgruppe, die gegebenenfalls durch ein- oder mehrfach durch Alkyl, z. B. Methyl, Halogen, eine geschützte Hydroxygruppe, Alkoxy, z. B. Methoxy, Alkanoyloxy, z. B. Acetoxy, eine geschützte Amino- oder Alkylaminogruppe, Alkanoylamino, z. B. Acetylamino oder Pivaloylamino, Alkoxycarbonyl-amino, z. B. t-Butoxycarbonylamino, Arylmethoxycarbonylamino, z.B. Benzyloxycarbonylamino, 9-Fluorenylmethoxycarbonyl und/oder Nitro substituiert sein können. Bevorzugt ist eine Substitution mit Alkyl oder Halogen, besonders bevorzugt eine mit Alkyl.

Der Ausdruck "Cycloalkyl" bezieht sich auf cyclische Alkylgruppen mit bevorzugt 3 bis 8 C-Atomen.

Der Ausdruck "Halogen" bezeichnet Fluor, Chlor, Brom und Jod.

Der Ausdruck "Heteroaryl" bezeichnet einen aromatischen fünf- oder sechs-gliedrigen Ring, der ein oder zwei Heteroatome ausgewählt aus Stickstoff, Sauerstoff oder Schwebel enthalten kann. Heteroarylringe können ein Substitutionsmuster aufweisen, wie es für "Aryl" definiert ist.

Der Ausdruck "Aminoschutzgruppe" bezieht sich auf aus dem Stand der Technik bekannte Schutzgruppen der Aminofunktion, wie sie zum Beispiel in der Peptidchemie eingesetzt werden. Beispiele für solche Aminoschutzgruppen sind die Benzyoxycarbonyl- oder die tert.B utoxycarbonyl-Schutzgruppe.

Als "reaktive Säurederivate" können Säurehalogenide eingesetzt werden, die von der Säure R¹-COOH abgeleitet sind, wobei R¹ Alkyl, Cycloalkyl, Heteroaryl oder Aryl ist. Bevorzugt sind Verbindungen, in denen R¹ Alkyl oder Aryl ist; besonders bevorzugt sind Verbindungen worin R¹ Alkyl ist. Dabei sind die entsprechenden Säurehalogenide (R¹-C(O)Hal), insbesondere die Chloride (die Säurechloride R¹-C(O)Cl), bevorzugt. Verzweigte aliphatische Carbonsäurehalogenide, wie z. B. 2-Ethylbuttersäurechlorid, Cyclohexancarbonsäurechlorid, 2,2-Dimethyl-propionsäurechlorid (Pivaloylchlorid) und Isovaleroylchlorid, finden auf Grund des sterischen und des positiven induktiven Effekts bevorzugt Verwendung.

Ausserdem können Chlorameisensäurealkylester, z. B. Chlorameisensäureethylester, verwendet werden. Die besonders bevorzugten reaktiven Säurederivate sind Pivaloylchlorid, Isovaleroylchlorid oder Chlorameisensäureethylester. Die entsprechenden Halogenide sind kommerziell erhältlich, bzw. nach bekannten Methoden herstellbar. Beispielsweise sind die entsprechenden Säurechloride durch Umsetzung der Säure mit Thionyl-, Phosphortrichlorid oder Phosphorpentachlorid erhältlich. Die erwähnten Chlorameisensäurealkylester sind kommerziell erhältlich bzw. nach bekannten Methoden herstellbar.

Als "Hilfsbasen" werden tertiäre Amine, aliphatische und aromatische, verwendet. Beispielsweise können Pyridin, N-Alkylmorpholine, z. B. N-Methyl- und N-Ethylmorpholin und Dialkylaniline, wie Dimethylanilin, eingesetzt werden. Bevorzugt ist Triethylamin. Die weitaus bevorzugtesten Basen sind tertiäre Amine wie N-Ethylmorpholin, Dimethylanilin, Triethylamin oder N,N,N',N'-Tetramethylethylendiamin, insbesondere aber Triethylamin.

Als Lösungsmittel können inerte Lösungsmittel wie Tetrahydrofuran, Toluol, Hexan, Aceton, Dioxan, bevorzugt niedere Carbonsäureester wie Alklylacetate, vor allem Methyl-, Ethyl- und Isopropylacetat, eingesetzt werden.

Vorteilhafterweise wird die Reaktion unter Temperaturkontrolle zwischen -25 und +25° C, bevorzugt zwischen 0 und +5° C, durchgeführt.

So umfasst die vorliegende Erfindung ebenfalls ein Verfahren zur Herstellung eines Anhydrids der Formel 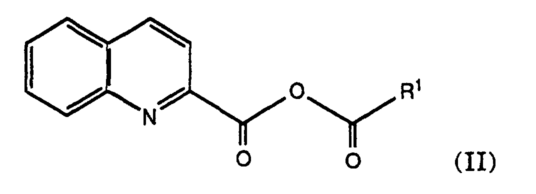 oder 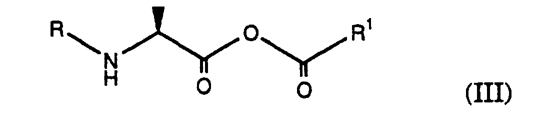 wobei R eine Aminoschutzgruppe und R¹ Alkyl, Cycloalkyl, Heteroaryl oder Aryl ist. Bevorzugt sind Verbindungen, in denen R¹ Alkyl oder Aryl ist; besonders bevorzugt sind Verbindungen worin R¹ Alkyl ist. Beispiele für diese Verbindungen sind zum Beispiel (S)-2-Benzyloxycarbonylaminopropionsäure-2,2-dimethyl-propionsäureanhydrid, (S)-2-tert-Butoxycarbonylamino-propionsäure-2,2-dimethylpropionsäureanhydrid und 2,2-Dimethyl-propionsäure-chinolin-2-carbonsäureanhydrid.

Das oben beschriebene Verfahren eignet sich beispielsweise für den Einsatz in der Peptidsynthese und/oder zur Herstellung pharmazeutisch wirksamer Substanzen bzw. entsprechender Ausgangs- oder Zwischenprodukte.

Damit umfasst die vorliegende Erfindung ein Verfahren zur Herstellung eines gemischten Chinolin-2-carbonsäureanhydrid der Formel 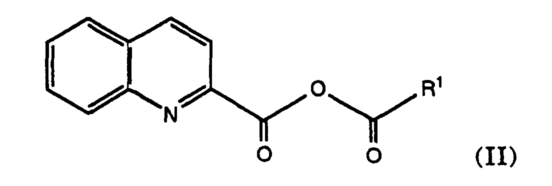 gemäss dem beschriebenen Verfahren, wobei R¹ wie oben definiert ist. Chinolin-2-carbonsäure wird dabei mit dem entsprechenden reaktiven Säurederivat, d. h. bevorzugt einem Säurehalogenid umgesetzt, das von der entsprechenden Säure R¹-COOH abgeleitet ist. Bevorzugt ist das entsprechende Säurechlorid.

Das erhaltene Anhydrid der obigen Formel (II) kann dann beispielsweise zu N-(2-Chinolylcarbonyl)-L-asparagin (Chinargin) durch Reaktion mit Asparagin umgesetzt werden. Chinargin ist an sich bekannt und beispielsweise in der Europäischen Patentanmeldung Nr. 611774 beschrieben. Es ist ein wertvolles Zwischenprodukt zur Herstellung pharmakologisch aktiver Verbindungen. So kann Chinargin wie in Beispiel 7 der genannten Europäischen Patentanmeldung dargestellt in pharmakologisch aktive Verbindungen überführt werden, die sich vor allem zur Behandlung viraler Infektionen eignen, insbesondere solcher Infektionen, die durch HIV oder andere Retroviren verursacht werden.

Zur Herstellung von Chinargin nach dem oben beschriebenen Verfahren kann Chinolin-2-carbonsäure mit einem reaktiven Derivat einer Säure R¹-COOH, wobei R¹ wie oben definiert ist, beispielsweise mit Pivaloylchlorid, und einer Hilfsbase, z. B. Triethylamin umgesetzt werden. Diese Umsetzung, die in Beispiel 1 im Detail beschrieben ist, liefert das entsprechende gemischte Anhydrid, hier z. B. 2,2-Dimethylpropionsäurechinolin-2-carbonsäureanhydrid, das nach Isolierung oder auch direkt, ohne weitere Aufreinigung, mit Asparagin in einer wässrigen, alkalischen Lösung zu Chinargin umgesetzt werden kann. Die Umsetzung mit Asparagin findet bevorzugt in einer wässrigen NaOH/NaHCO₃-Lösung statt. Mit diesem Verfahren lässt sich N-(2-Chinolylcarbonyl)-L-asparagin (S-Chinargin) ohne die Isolierung eines Zwischenproduktes direkt aus den Edukten mit hoher Ausbeute herstellen.

Weiterhin umfasst die vorliegende Erfindung die Herstellung pharmazeutisch aktiver Substanzen. Beispielsweise kann das obengenannte N-(2-Chinolylcarbonyl)-L-asparagin durch Umsetzung mit 2-[3(S)-Amino-2(R)-hydroxy-4-phenylbutyl]-N-tert.butyl-decahydro-(4aS,8aS)-isochinolin-3(S)carboxamid, das aus der Europäischen Patentanmeldung Nr. 635,493 bekannt ist, in Gegenwart eines Kupplungsreagenzes wie z. B. eines Carbodiimids und einer N-Hydroxyverbindung, wobei die N-Hydroxyverbindung in katalytischer Menge vorliegt, umgesetzt werden. Wie in Beispiel 7 der Europäischen Patentanmeldung Nr. 611774 beschrieben, können in Gegenwart von Dicyclohexylcarbodiimid mittels einer katalytischen Menge 1-Hydroxy-2(1H)-pyridon in einem inertem Lösungsmittel bzw. Lösungsmittelgemisch wie Ethylacetat/Tetrahydrofuran die obengenannten Substanzen zu N-t-Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid oder zu davon abgeleiteten, pharmazeutisch geeigneten Salzen oder zu entsprechenden Estern umgesetzt werden.

Damit umfasst die Erfindung auch ein Verfahren zur Herstellung dieser Verbindungen. Ein solches Verfahren umfasst in einem ersten Schritt die Herstellung eines gemischten Anhydrids und seine Umsetzung zu Chinargin wie oben beschrieben. Das gemischte Anhydrid, z. B. 2,2-Dimethylpropionsäure-chinolin-2-carbonsäureanhydrid, kann durch Zugabe einer Hilfsbase, z. B. Triethylamin, zu einem Gemisch aus Chinolin-2-carbonsäure und einem reaktiven Säurederivat, bevorzugt Pivaloylchlorid, erhalten werden. Das entstandene gemischte Anhydrid wird anschliessend mit L-Asparagin in alkalischer Lösung zu N-(2-Chinolylcarbonyl)-L-asparagin umgesetzt. Diese Substanz wird dann in einem Folgeschritt mit 2-[3(S)-Amino-2(R)-hydroxy-4-phenylbutyl]-N-tert.butyl-decahydro-(4aS,8aS)-isochinolin-3(S)carboxamid zu N-t-Butyldecahydro-2-[2(R)-hydroxy-4-phenyl-3(S)-[[N-(2-chinolylcarbonyl)-L-asparaginyl]-amino]butyl]-(4aS,8aS)-isochinolin-3(S)-carboxamid wie oben beschrieben oder gegebenenfalls zu einem entsprechenden Salz, wobei das entsprechende Methansulfonsäuresalz bevorzugt ist, oder zu einem Ester umgesetzt.

Weiterhin ist es mittels des erfindungsgemässen Verfahrens möglich eine Säure der Formel (I) 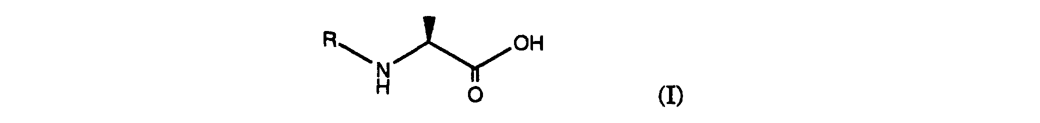 mit einem reaktiven Derivat einer Säure R¹-COOH zu einem Anhydrid der Formel (III) 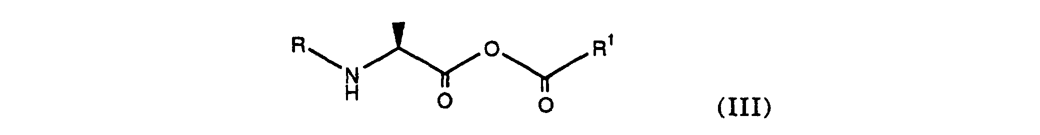 umzusetzen, wobei R eine Aminoschutzgruppe wie oben definiert und R¹ Alkyl, Cycloalkyl, Heteroaryl oder Aryl ist. Bevorzugt sind Verfahren in denen Verbindungen eingesetzt werden, in denen R¹ Alkyl oder Aryl ist; besonders bevorzugt sind Verbindungen worin R¹ Alkyl ist. Die Aminoschutzgruppe kann eine aus dem Stand der Technik bekannte Schutzgruppe sein, wie sie zum Beispiel in der Peptidchemie eingesetzt werden. Beispiele für solche Aminoschutzgruppen sind die Benzyoxycarbonyl- oder die tert.Butoxycarbonyl-Schutzgruppe.

So ist es mit dem erfindungsgemässen Verfahren möglich, (S)-[1-(2-Benzyloxycarbonylamino-propionyl)-piperidin-4-yloxy]-essigsäureetylester und (S)-[1-(2-tert-Butyloxycarbonylamino-propionyl)-piperidin-4-yloxy]-essigsäureethylester als weitere wichtige pharmazeutische Zwischenprodukte mittels des oben beschriebenen Verfahrens herzustellen. Wie in den Beispielen 2 und 3 beschrieben, kann man auch in diesen Fällen sogar ohne Isolierung der entsprechenden gemischten Anhydride die entsprechenden Zwischenprodukte in sehr guter Ausbeute erhalten. Im einzelnen erfolgt die Reaktion durch Umsetzung einer geschützten Aminopropionsäure z. B. (S)-2-Benzyloxy-carbonylamino-propionsäure bzw. (S)-2-tert-Butyloxycarbonylamino-propionsäure, mit einem wie oben definierten, reaktiven Säurederivat, hier in beiden Fällen mit Pivaloylchlorid, unter Verwendung eines tertiären Amins, hier Triethylamin, als Hilfsbase. Bei der Aminoschutzgruppe der Aminopropionsäure kann es sich um jede geeignete Aminoschutzgruppe handeln, bevorzugt sind die Benzyloxycarbonyl- und die tert.-Butyloxycarbonylschutzgruppe. Die erhaltenen gemischten Anhydride (S)-2-Benzyloxycarbonyl-aminopropionsäure-2,2-dimethylpropionsäureanhydrid und (S)-2-tert.-Butoxycarbonylamino-propionsäure-2,2-dimethylpropion-säureanhydrid können mit (Piperidin-4-yloxy)-essigsäureethylester, z. B. in einer Kaliumphosphat-gepufferten, wässrigen Ethylacetatsuspension umgesetzt werden, wobei man die obengenannten Zwischenprodukte erhält. Diese Zwischenprodukte können zu pharmazeutischen Wirkstoffen weiterverarbeitet werden.

Beispielsweise ist es mit dem erfindungsgemässen Verfahren möglich, diese Zwischenprodukte zur Herstellung von [Z]-(S)-[[1-(2-[[4-(Amino-Hydroximino-methyl)-benzoyl]amino]-1-oxopropyl]-4-piperidinyl]oxy]essigsäureethylester, einem Fibrinogen Rezeptorantagonisten zu verwenden (Alig et al. (1992) J. Med. Chem. 35, 4393 - 4407; Weller et al. (1996) J. Med. Chem. 39, 3139-3147). Zur Herstellung dieser Substanz kann das oben beschriebene Verfahren folgendermassen weitergeführt werden: Die Schutzgruppen der oben beschriebenen Zwischenprodukte können abgespalten und das erhaltene Amin mit einem entsprechenden Säurechlorid und nachfolgender Umsetzung mit Hydroxylamin zu der oben genannten Substanz umgesetzt werden. Im einzelnen kann die Benzyloxycarbonylschutzgruppe durch Hydrierung und die tert.-Butoxy-carbonylschutzgruppe durch Säure aus (S)-[1-(2-Benzyloxycarbonylamino-propionyl)-piperidin-4-yloxy]-essigsäureetylester und (S)-[1-(2-tert-Butyloxycarbonylamino-propionyl)-piperidin-4-yloxy]-essigsäureethylester abgespalten werden. Das freigesetzte Amin kann dann mit 4-Cyanobenzoe-säurechlorid, das aus Thionylchlorid und 4-Cyanobenzoesäure herstellbar ist, und nachfolgend durch Umsetzung mit Hydroxylaminhydrochlorid und Triethylamin und Aufarbeitung im saurem Medium zu [Z]-(S)-[[1-[2-[[4-(Amino-Hydroximino-methyl)-benzoyl]amino]-1-oxopropyl]-4-piperidinyl]oxy]essigsäure-ethylester gemäss Beispiel 4 umgesetzt werden.

Folglich bezieht sich das erfindungsgemässe Verfahren auch auf die Herstellung von [Z]-(S)-[[1-[2-[[4-(Amino-hydroximino-methyl)-benzoyl]amino]-1-oxopropyl]-4-piperidinyl]oxy]essigsäureethylester, dadurch gekennzeichnet, dass in einem ersten Schritt ein gemischtes Anhydrid wie oben beschrieben hergestellt wird und durch Reaktion mit einer Verbindung der Formel (IV), 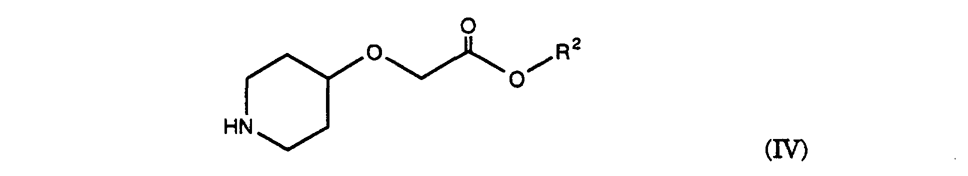 wobei R² eine Alkylgruppe, bevorzugt Ethyl oder tert.Butyl, darstellt, zu einem Ester der Formel (V) umgesetzt wird, 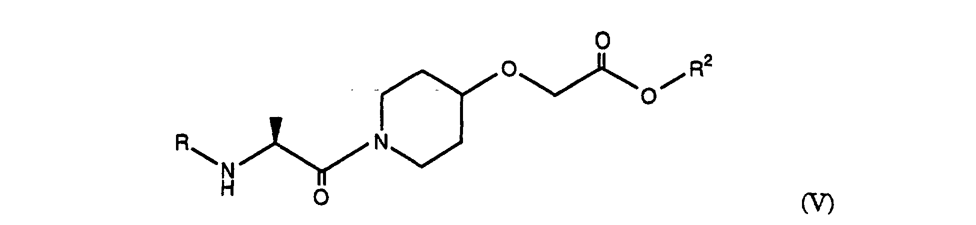 wobei R und R² wie oben definiert sind. Nachfolgend kann die Aminoschutzgruppe R abgespalten und gegebenenfalls die Gruppe R² zum Ethylester umgeestert werden, wobei die Abspaltung der Aminoschutzgruppe und die Umesterung zum Ethylester je nach verwendeter Schutzgruppe gegebenenfalls gleichzeitig, z. B. im Fall der tert. Butyl-Schutzgruppe durch Umsetzung mit H₂SO₄/Ethanol, stattfinden kann. Anschliessend kann das erhaltene Amin der Formel (VI), 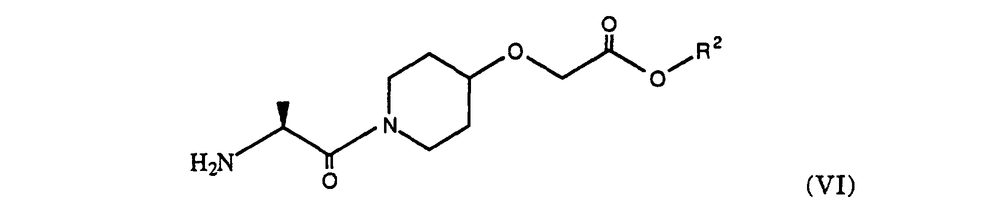 wobei R² wie oben definiert ist, mit 4-Cyanobenzoesäurechlorid zu der Verbindung der Formel (VII) umgesetzt werden, 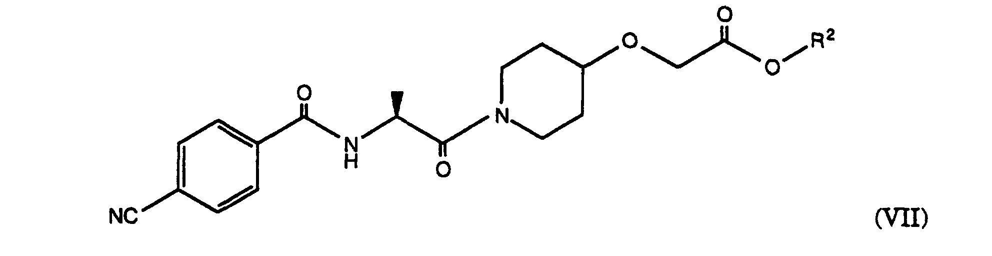 wobei die Gruppe R² wie oben definiert ist. Als Alternative kann bei der Verwendung von z. B. der Benzyloxycarbonyl-Schutzgruppe die Abspaltung der Schutzgruppe durch Hydrierung erfolgen, gefolgt von der Umsetzung mit 4-Cyanobenzoesäurechlorid und der Umesterung zur entsprechenden Ethylesterverbindung (R² = Ethyl). Gegebenenfalls kann natürlich auch, wie in Beispiel 4 beschrieben, über die Verbindung (IV) mit R² = Ethyl, gleich der entsprechende Ethylester eingeführt werden. Anschliessend kann [Z]-(S)-[[1-[2-[[4-(Amino-hydroximino-methyl)-benzoyl]amino]-1-oxopropyl]-4-piperidinyl]oxy]essigsäureethylester durch Reaktion von Hydroxylaminhydrochlorid mit der Verbindung der Formel (VII) erhalten werden. Gegebenenfalls kann die erhaltene Verbindung in ein pharmazeutisch geeignetes Salz überführt werden.

Die folgenden Beispiele dienen der Erläuterung der Erfindung.

### BEISPIELE

### Beispiel 1: Herstellung von N-(2-Chinolylcarbonyl)-L-asparagin (S-Chinargin)

34.60 g (200 mmol) Chinolin-2-carbonsäure (Benechim S.A.) wurde in 350 ml Ethylacetat (Fluka) unter Rühren und unter Schutzgas bei 0 bis 5° C suspendiert und mit 25.0 ml (200 mmol) Pivaloylchlorid (Fluka) versetzt. Zu der weissen Suspension wurden innerhalb von 30 Minuten 28.0 ml (200 mmol) Triethylamin (Fluka) gegeben, wobei die Temperatur bei 0 bis 5° C gehalten wurde. Anschliessend wurde die Suspension für 30 Minuten bei 0 bis 5° C gerührt. Die weisse Suspension wurde filtriert und der Filterkuchen mit 100 ml gekühltem (0 - 5° C) Ethylacetat (Fluka) gewaschen. Die Filtrate enthielten das entstandene 2,2-Dimethylpropionsäure-chinolin-2-carbonsäureanhydrid mit den folgenden Substanzcharakteristika: Schmelzpunkt: 47° C; MS: [M+H]⁺ 258; IR: 1750 cm⁻¹, 1775 cm⁻¹. Die gekühlten (0 - 5° C), schwach gelben Filtrate wurden vereinigt und innerhalb von 5 Minuten unter intensivem Rühren mit einer vorgekühlten Lösung aus 30.0 g (200 mmol) L-Asparagin (Fluka), 8.0 g (200 mmol) Natriumhydroxid (Fluka) und 16.80 g (200 mmol) Natriumbicarbonat (Fluka) in 350 ml kaltem, deinosiertem Wasser (0 - 5° C) versetzt, wobei die Temperatur zwischen 0 und 5° C gehalten wurde. Anschliessend wurde die Kühlung entfernt und das schwach grüne Zweiphasensystem für ca. 17 Stunden bei Raumtemperatur gerührt. Die wässrige Phase des nun farblosen Zweiphasensystems wurde unter Rühren und unter Argon mit 200 ml Methanol (Merck) versetzt. Dann wurden tropfenweise 49 ml 25% Salzsäure (Merck) bis pH 3.0 zugegeben. Dabei erhöhte sich die Temperatur auf 28° C und Kristallisation setzte ein. Die weisse Suspension wurde für eine Stunde bei Raumtemperatur gerührt, anschliessend auf 0 - 5° C abgekühlt und für eine weitere Stunde bei dieser Temperatur gerührt. Der abfiltrierte Filterkuchen wurde portionsweise mit insgesamt 80 ml kaltem, deionisiertem Wasser (0 - 5° C) gewaschen und in einem Rotationsverdampfer bei 50° C/10 mbar für 6 Stunden getrocknet. Ausbeute: 47.52 g (83%) N-(2-Chinolylcarbonyl)-L-asparagin in Form weisser Kristalle [Schmelzpunkt: 209° (Zersetzung), HPLC-Analyse 99.8% (Fläche)].

### Beispiel 2: Herstellung von (S)-[1-(2-Benzyloxycarbonylaminopropionyl)-piperidin -4-yloxy]-essigsäureethylester

55.8 g (250 mmol) (S)-2-Benzyloxycarbonylamino-propionsäure (herstellbar aus der entsprechenden Aminocarbonsäure durch Schutz der Aminofunktion mit einem Benzyloxycarbonylderivat; Bodansky et al., loc. cit.) wurden unter Rühren und unter Argon in 560 ml Ethylacetat (Fluka) gelöst, auf 0 bis 5° C abgekühlt und mit 31.0 ml (250 mmol) Pivaloylchlorid (Fluka) versetzt. Zu der leicht gelblichen Lösung wurde innerhalb von 30 Minuten tropfenweise 35.0 ml (Fluka) Triethylamin (Fluka) zugegeben, wobei die Temperatur bei 0 bis 5° C gehalten wurde. Es bildete sich eine weisse Suspension. Das weisse Präzipitat wurde durch einen vorgekühlten Glasfilter (G3) filtriert und der Filterkuchen mit 140 ml kalten Ethylacetat (0 bis 5° C, Fluka) gewaschen. Zu den vereinigten Filtraten, eine klare, farblose Flüssigkeit, die das Anhydrid enthielt, wurden 55.9 g (250 mmol) Piperidin-4-yloxy-essigsäureethylester-hydrochlorid (herstellbar aus der entsprechenden tert.-Butylverbindung (Alig et al., loc. cit.) durch Umesterung mit Aethanol/Salzsäure) und 66.6 g (250 mmol) Na₃PO₄·3H₂O zugegeben. Die weisse Suspension wurde bei 5 bis 10° C unter Argon und unter heftigem Rühren innerhalb von 30 Sekunden mit 500 ml deionisiertem Wasser versetzt, wobei die Temperatur auf 20° C anstieg. Das farblose Zweiphasensystem wurde für eine Stunde bei 20 bis 25° C kräftig gerührt. Anschliessend wurde die wässrige Phase abgetrennt und die organische Phase mit 1 M Natriumbicarbonatlösung und dann mit 1 N wässriger Ammoniaklösung gewaschen. Die organische Phase wurde über ca. 50 g Natriumsulfat getrocknet, filtriert und der Filterkuchen mit ca. 100 ml Ethylacetat gewaschen. Die vereinigten Filtrate wurden in einem Rotationsverdampfer eingedampft und der ölige Rückstand mit 100 ml Ethanol aufgenommen. Die klare Lösung wurde bei 50°/20 mbar eingedampft, um 90.2 g (92 Gew.%) (S)-[1-(2-Benzyloxycarbonylaminopropionyl)-piperidin-4-yloxy]-essigsäureethylester als klares, farbloses Öls zu ergeben.

### Beispiel 3: Herstellung von (S )-[1-(2 -tert-Butyloxycarbonylaminopropionyl)-piperidin-4-yloxy]-essigsäureethylester

47.3 g (250 mmol) (S)-2-tert-Butyloxycarbonylaminopropionsäure (Fluka) wurden in 560 ml Ethylacetat (Fluka) unter Rühren und unter Argon gelöst, auf 0 - 5° C abgekühlt und mit 31.0 ml (250 mmol) Pivaloylchlorid (d = 0.98; Fluka) versetzt. Die leicht gelbliche Lösung wurde innerhalb von 30 Minuten tropfenweise mit 35.0 ml (250 mmol) Triethylamin (d = 0.726; Fluka) versetzt, wobei die Temperatur im Bereich von 0 bis 5° C gehalten wurde. Es bildete sich eine weisse Suspension. Das weisse Präzipitat wurde durch einen vorgekühlten, gesinterten Glasfilter (G3) abfiltriert und der Filterkuchen mit 140 ml kaltem Etylacetat (0 bis 5° C) gewaschen. Zu den vereinigten Filtraten, eine schwach gelbe Lösung, wurden 55.9 g (250 mmol) Piperidin-4-yloxy)-essigsäureethylesterhydrochlorid und 66.6 g K₃PO₄·3H₂O (250 mmol; Merck) zugegeben. Die weisse Suspension wurde bei 5 bis 10° C unter Argon und starkem Rühren innerhalb von 30 Sekunden mit 500 ml deionisiertem Wasser versetzt, wobei die Temperatur auf 20° C anstieg. Das farblose Zweiphasensystem wurde für 2 Stunden bei 20 bis 25° C gerührt. Die wässrige Phase wurde abgetrennt und die organische Phase mit 500 ml einer 1 M Natriumbicarbonatlösung gewaschen. Die organische Phase wurde über ca. 50 g Natriumsulfat getrocknet, filtriert und der Filterkuchen mit ca. 100 ml Ethylacetat gewaschen. Die vereinigten Filtrate wurden im Rotationsverdampfer bei 50°/20 mbar eingedampft, wobei sich 85.5 g (95 Gew.%) (S)-[1-(2-tert-Butyloxycarbonylaminopropionyl)-piperidin-4-yloxy]-essigsäureethylester als Rohprodukt in Form eines klaren, gelblichen Öls ergaben (HPLC Analyse: 88.9 % (Fläche).

### Beispiel 4: Herstellung von [Z]-(S)-[[1-[2-[[4-(Amino-Hydroximino-methyl)-benzoyl]amino]-1-oxopropyl]-4-piperidinyl]oxy]essigsäureethylester

Zur Herstellung der obengenannten Verbindung wurden 90,2 g (230 mmol) (S)-[1-(2-Benzyloxycarbonylamino-propionyl)-piperidin-4-yloxy]-essigsäureethylester in 450 ml Ethanol mit 4,5 g 10% Palladium/Kohle versetzt und die resultierende Lösung unter intensivem Rühren bei einem Wasserstoffdruck von 1,1 bar bei 22 bis 24° C für 40 Minuten hydriert. Nach Evakuierung wurde der Hydrierkessel wieder gefüllt und für weitere 30 Minuten gerührt. Die Suspension wurde anschliessend über 30 g Speedex (Dicalite Speedex) und der erhaltene Filterkuchen mit 450 ml Ethanol gewaschen und die vereinigten, klaren Filtrate auf 0 bis 5° C abgekühlt und mit 32 ml Triethylamin (230 mmol) versetzt. Zu dieser Lösung wurde unter Rühren eine leicht gelbe Lösung von 38 g (230 mmol) 4-Cyanobenzoesäurechlorid in 80 ml Toluol zugegeben, wobei die Temperatur zwischen 0 und 5° C gehalten wurde. Die leicht gelbe Lösung wurde bei Raumtemperatur für eine Stunde gerührt und dann mit 31,9 g (460 mmol) Hydroxylaminhydrochlorid (Fluka) und mit 64 ml (460 mmol) Triethylamin (Fluka) versetzt, wobei die Temperatur auf ca. 28°C anstieg. Die weisse Suspension wurde bei Raumtemperatur für 17 Stunden bei 20 bis 25°C gerührt, wobei sich nach einer Stunde eine fast klare Suspension bildete, gefolgt von der Bildung eines weissen Niederschlags. Die weisse Suspension wurde mit 36 ml einer 25% Salzsäure versetzt. Die Suspension wurde auf 0 bis 5° C gekühlt, für eine Stunde gerührt, durch einen vorgekühlten gesinterten Glasfilter (G3) filtriert und der Filterkuchen mit 250 ml kaltem Ethanol gewaschen. Anschliessend wurde bis zur Gewichtskonstanz getrocknet, wobei sich 70,6 g (= 73 Gew%) [Z]-(S)-[[1-[2-[[4-(Amino-hydroximino-methyl)-benzoyl]amino]-1-oxopropyl]-4-piperidinyl]oxy]essigsäureethylester ergaben. Das Pulver wurde unter Rückfluss und unter Rühren in einer Mischung aus 560 ml Ethanol und 140 ml Wasser gelöst und anschliessend die Lösung innerhalb von zwei Stunden auf Raumtemperatur abgekühlt und anschliessend für eine Stunde bei einer Temperatur von 0 bis 5° C gerührt. Das entstandene Filtrat wurde durch einen vorgekühlten Glasfilter (G3) filtriert und der Filterkuchen mit einer Mischung aus 140 ml kaltem Ethanol (0 bis 5° C) und 35 ml kaltem Wasser (0 bis 5°) gewaschen; anschliessend wurde das Produkt bei 50°/20 mbar zur Gewichtskonstanz getrocknet. Die erhaltenen weissen Kristalle (65,6 g; 68 Gew.%) wurden in einer Mischung aus 528 ml Ethanol und 132 ml Wasser suspendiert und mit 3,3 g Aktivkohle (Norit SX-1) behandelt. Die schwarze Suspension wurde unter Erhitzen und Rückfluss für 15 Minuten erhitzt, auf 65 bis 70° C abgekühlt und über einen Glasfilter auf einem vorgewärmten Trichter gegeben. Die Filtrate wurden gerührt, wobei ab 60° C ein Niederschlag ausfiel. Die weisse Suspension wurde innerhalb von drei Stunden auf Raumtemperatur, dann auf 0 bis 5° C abgekühlt und nach einstündigem Rühren durch einen entsprechend vorgekühlten gesinterten Glasfilter (G3) filtriert. Der Filterkuchen wurde mit einer Mischung aus 132 ml kaltem Ethanol und 33 ml Wasser (0 bis 5° C) gewaschen und anschliessend bei 50°/20 mbar zur Gewichtskonstanz getrocknet. Ausbeute: 60,15 g (55%); Smp: 212-213°; ee (HPLC) >99,9%; Opt. Rot. [α]₃₆₅ +223,5 (c = 1,0; HCl).

### Beispiel 5: Herstellung weiterer gemischter Anhydride

Analog zu den Beispielen 1 bis 3 lassen sich nach dem erfindungsgemässen Verfahren weitere Verbindung herstellen. In der folgenden Tabelle sind die Ergebnisse weiterer Reaktionen zusammengefasst.

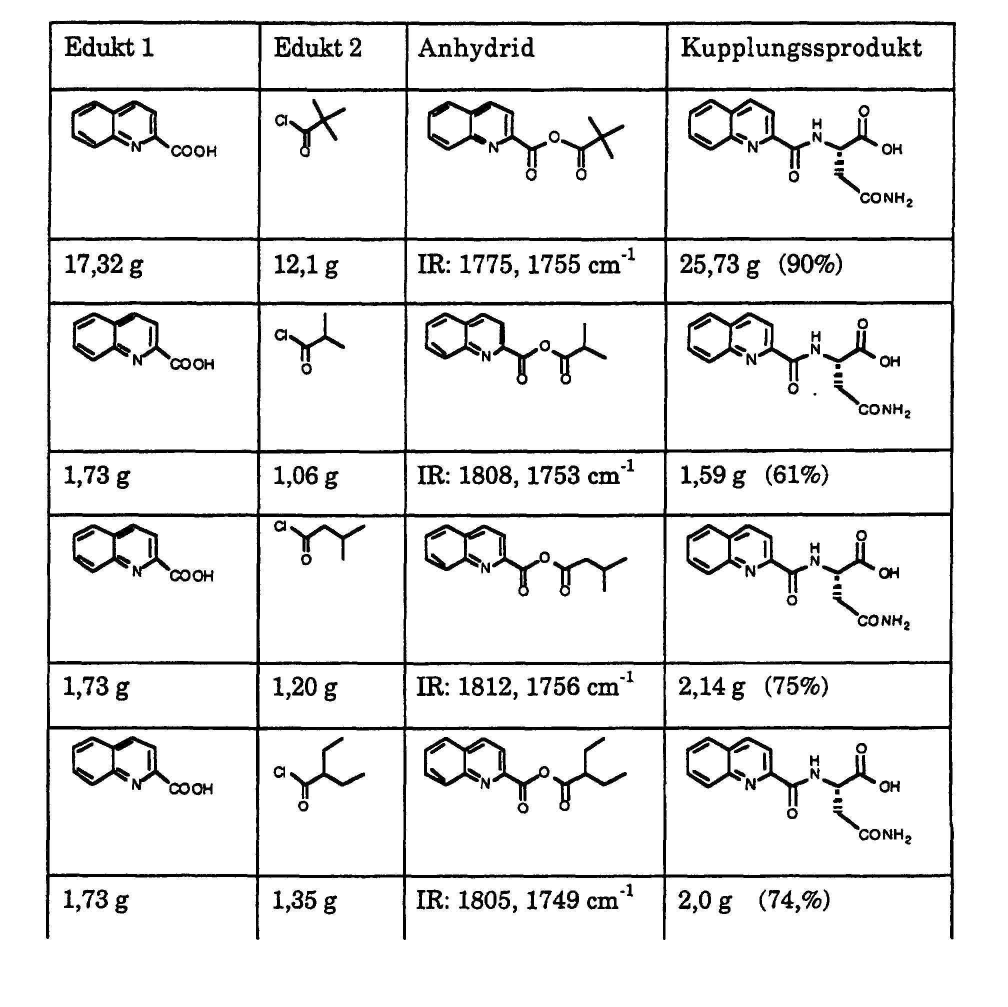

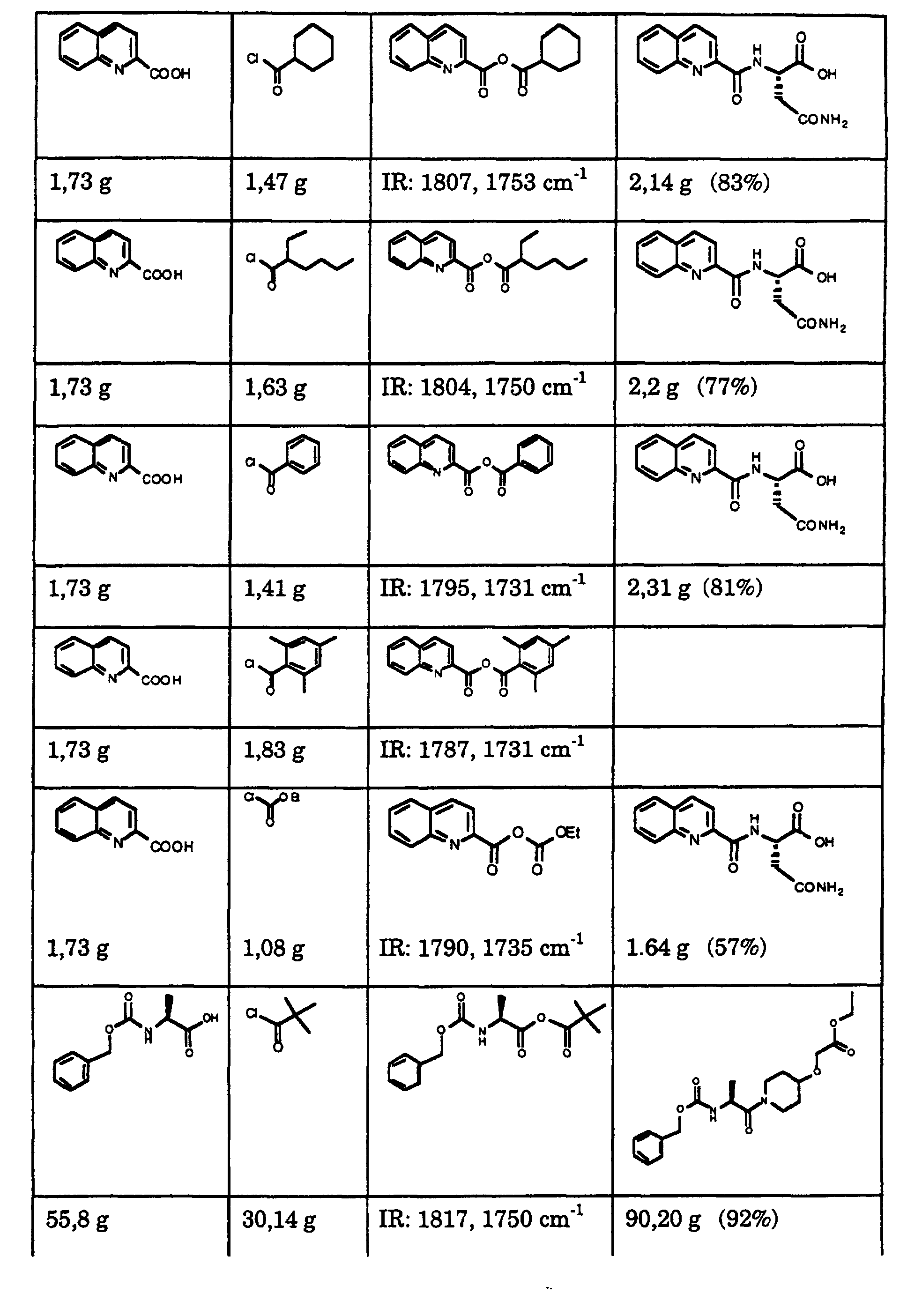

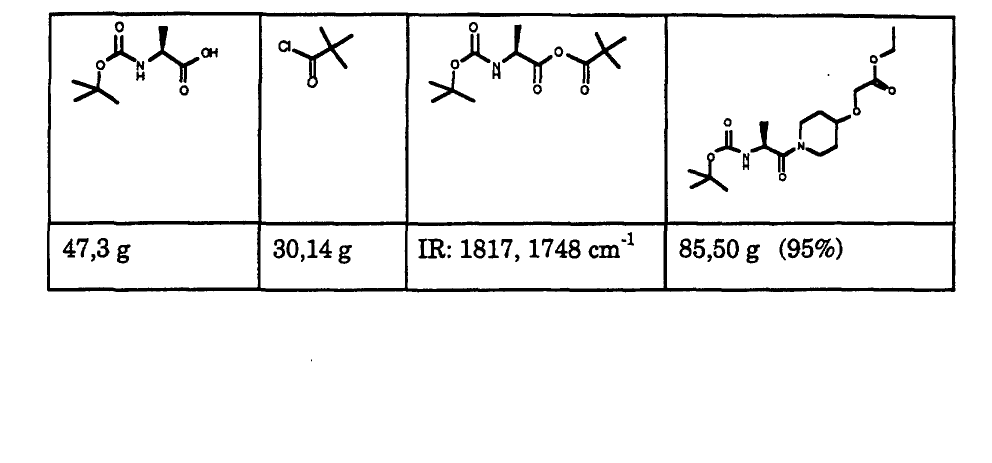

## Patentansprüche

1. Verfahren zur Herstellung gemischter Anhydride, **dadurch gekennzeichnet, dass** eine Hilfsbase zum Gemisch aus Säure und reaktivem Säurederivat gegeben wird, worin die Säure eine Carbonsäure ist, die Hilfsbase ein tertiäres Amin ist und das reaktive Säurederivat ein Säurehalogenid einer Säure R¹-COOH oder ein Chlorameisensäurealkylester ist,
worin R¹ Alkyl, Cycloalkyl, Heteroaryl oder Aryl ist.

2. Verfahren gemäss Anspruch 1, **dadurch gekennzeichnet, dass** das reaktive Säurederivat Pivaloylchlorid, Isovaleroylchlorid oder Chlorameisensäureethylester eingesetzt wird.

3. Verfahren gemäss Anspruch 1, **dadurch gekennzeichnet, dass** als Säure eine Alkancarbonsäure oder eine geschützte Aminocarbonsäure eingesetzt wird.

4. Verfahren gemäss einem der Ansprüche 1 bis 2, **dadurch gekennzeichnet**, das Chinolin-2-carbonsäure mit einem reaktiven Derivat einer Säure R¹-COOH zu dem Chinolin-2-carbonsäureanhydrid der Formel (II) 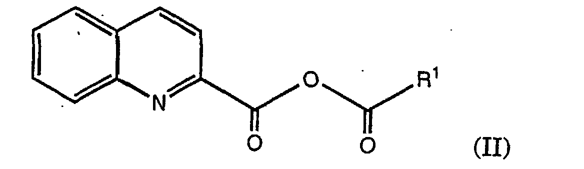 umgesetzt wird, wobei R¹ Alkyl, Cycloalkyl, Heteroaryl oder Aryl ist.

5. Verfahren gemäss Anspruch 4, **dadurch gekennzeichnet**, das das erhaltene Chinolin-2-carbonsäureanhydrid der Formel (II) mit Asparagin zu Chinargin umgesetzt wird.

6. Verfahren gemäss Anspruch 5, **dadurch gekennzeichnet, dass** das Chinolin-2-carbonsäureanhydrid der Formel (II) mit L-Asparagin zu N-(2-Chinolylcarbonyl)-L-asparagin und anschliessend mit 2-[3(S)-Amino-2(R)-hydroxy-4-phenylbutyl]-N-tert.butyl-decahydro-(4aS,8aS)-isochinolin-3(S)carboxamid umgesetzt wird und gegebenenfalls das erhaltene N-t-Butyldecahydro-2-[2(R)-hydroxy-4-phenyl-3(S)-[[N-(2-chinolylcarbonyl)-Lasparaginyl]amino]butyl]-(4aS,8aS)-isochinolin-3(S)-carboxamid in ein pharmazeutisch geeignetes Salz oder in einen pharmazeutisch geeigneten Ester überführt wird.

7. Verfahren gemäss einem der Ansprüche 1 bis 35 **dadurch gekennzeichnet**, das eine Säure der Formel (I) 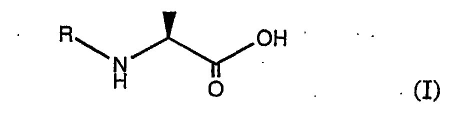 wobei R eine Aminoschutzgruppe darstellt, mit einem reaktiven Derivat einer Säure R¹-COOH zu Anhydrid der Formel (III) 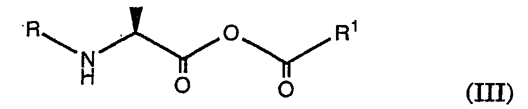 umgesetzt wird, wobei R die oben angegebene Bedeutung besitzt und R¹ Alkyl, Cycloalkyl, Heteroaryl oder Aryl ist.

8. Verfahren gemäss Anspruch 7 zur Herstellung von (S)-2-Benzyloxycarbonylamino-propionsäure-2,2-dimethyl-propionsäureanhydrid oder (S)-2-tert-Butoxycarbonylamino-propionsäure-2,2-dimethylpropionsäureanhydrid durch Umsetzung von (S)-2-Benzyloxy-carbonylaminopropionsäure oder (S)-2-tert-Butyloxycarbonylaminopropionsäure einem reaktiven Säurederivat der 2,2-Dimethyl-propionsäure.

9. Verfahren gemäss Anspruch 7 oder 8, **dadurch gekennzeichnet, dass** das Anhydrid
a) durch Reaktion mit einer Verbindung der Formel (IV), 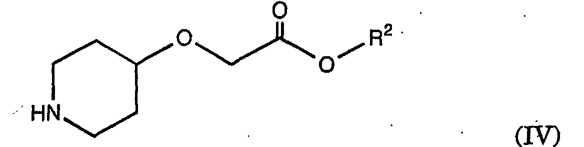 wobei R² eine Alkylgruppe darstellt,
zu einem Ester der Formel (V) umgesetzt wird, 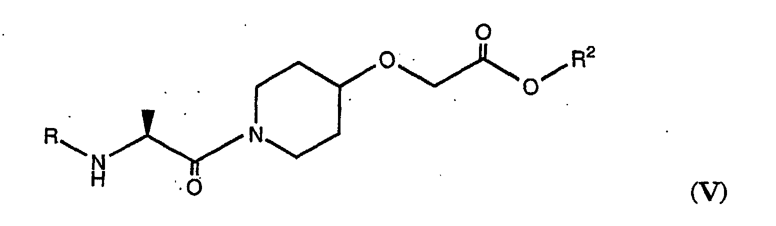 wobei R eine Aminoschutzgruppe darstellt, und R² wie oben definiert ist,
b) die Aminoschutzgruppe R abgespalten, und
c) gegebenenfalls die Gruppe R² zum Ethylester umgeestert wird, wobei die Reaktionsschritte b) und c) gegebenenfalls gleichzeitig stattfinden können und anschliessend
d) das erhaltene Amin der Formel (VI) 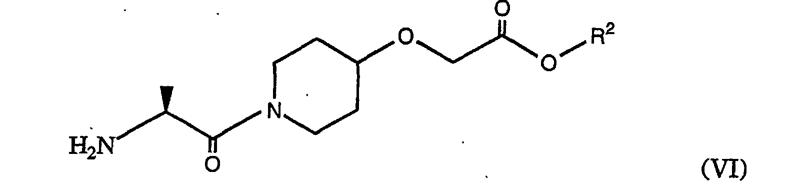 wobei R¹ wie oben definiert ist, mit 4-Cyanobenzoesäurechlorid zu der Verbindung der Formel (VII) umgesetzt wird, 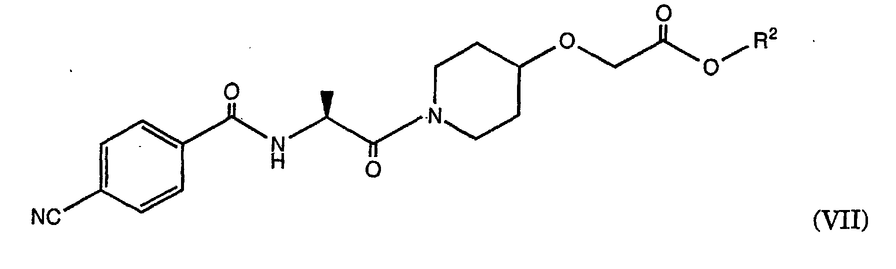 wobei die Gruppe R² wie oben definiert ist, und anschliessend gegebenenfalls R² zum Ethylester umgeestert wird, anschliessend
e) [Z]-(S)-[[1-[2-[[4-(Amino-hydroximino-methyl)-benzoyl]amino]-1-oxopropyl]-4-piperidinyl]oxy]essigsäureethylester durch Reaktion von Hydroxylaminhydrochlorid mit der Verbindung der Formel (VII) erhalten wird und
f) der erhaltene [Z]-(S)-[[1-[2-[[4-(Amino-hydroximino-methyl)-benzoyl]amino]-1-oxopropyl]-4-piperidinyl]oxy]essigsäureethylester gegebenenfalls in ein pharmazeutisch geeignetes Salz überführt wird.

10. Verwendung eines Verfahrens gemäss einem der Ansprüche 1 bis 9 zur Herstellung von N-t-Butyl-decahydro-2-[2(R)-hydroxy-4-phenyl-3(S)-[[N-(2-chinolylcarbonyl)-L-asparaginyl] amino]butyl]-(4aS,8aS)-isochinolin-3(S)-carboxamid oder [Z]-(S)-[[1-[2-[[4-(Amino-Hydroximino-methyl)-benzoyl]amino]-1-oxopropyl]-4-piperidinyl]oxy]essigsäureethylester oder davon abgeleiteter Salze oder Ester.

## Claims

1. A process for the manufacture of mixed anhydrides, **characterized by** adding an adjuvant base to a mixture of acid and reactive acid derivative, wherein the acid is a carboxylic acid, the adjuvant base is a tertiary amine and the reactive acid derivative is an acid halide of an acid R¹-COOH or an alkyl chloroformate in which R¹ is alkyl, cycloalkyl, heteroaryl or aryl.

2. A process according to claim 1, **characterized in that** the reactive acid derivative is pivaloyl chloride, isovaleroyl chloride or ethyl chloroformate.

3. A process according to claim 1, **characterized in that** an alkanecarboxylic acid or a protected aminocarboxylic acid is used as the acid.

4. A process according to one of claims 1 to 2, **characterized in that** quinoline-2-carboxylic acid is converted with a reactive derivative of an acid R¹-COOH into the quinoline-2-carboxylic acid anhydride of formula (II) 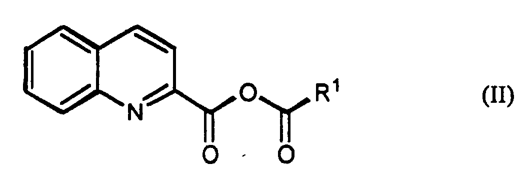 wherein R¹ is alkyl, cycloalkyl, heteroaryl or aryl.

5. A process according to claim 4, **characterized in that** the quinoline-2-carboxylic acid anhydride of formula (II) obtained is converted with asparagine into quinargine.

6. A process according to claim 5, **characterized in that** the quinoline-2-carboxylic acid anhydride of formula (II) is converted with L-asparagine into N-(2-quinolylcarbonyl)-L-asparagine and subsequently reacted with 2-[3(S)-amino-2(R)-hydroxy-4-phenylbutyl]-N-tert.butyl-decahydro-(4aS,8aS)-isoquinoline-3(S)-carboxamide and, if desired, the N-t-butyl-decahydro-2-[2(R)-hydroxy-4-phenyl-3(S)- [[N-(2-quinolylcarbonyl)-L-asparaginyl]amino]-butyl]-(4aS,8aS)-isoquinoline-(3S)-carboxamide obtained is converted into a pharmaceutically suitable salt or into a pharmaceutically suitable ester.

7. A process according to any one of claims 1 to 3, **characterized in that** an acid of formula (I) 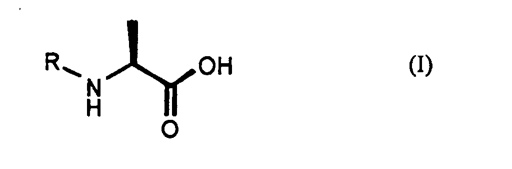 wherein R represents an amino protecting group,
is converted with a reactive derivative of an acid R¹-COOH into an anhydride of formula (III) 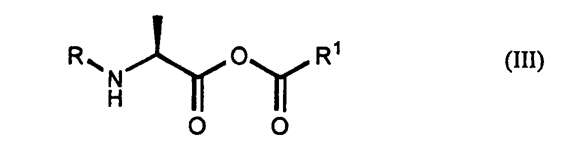 wherein R has the significance given above and R¹ is alkyl, cycloalkyl, heteroaryl or aryl.

8. A process according to claim 7 for the manufacture of (S)-2-benzyloxycarbonylamino-propionic acid-2,2-dimethyl-propionic acid anhydride or (S)-2-tert-butoxycarbonylamino-propionic acid-2,2-dimethyl-propionic acid anhydride by reaction of (S)-2-benzyloxycarbonylamino-propionic acid or (S)-2-tert-butyloxycarbonylaminopropionic acid with a reactive acid derivative of 2,2-dimethyl-propionic acid.

9. A process according to claim 7 or 8, **characterized in that** the anhydride
a) is converted by reaction with a compound of formula (IV) 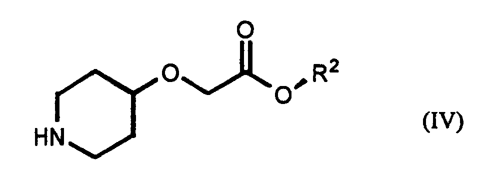 wherein R² represents an alkyl group,
into an ester of formula (V) 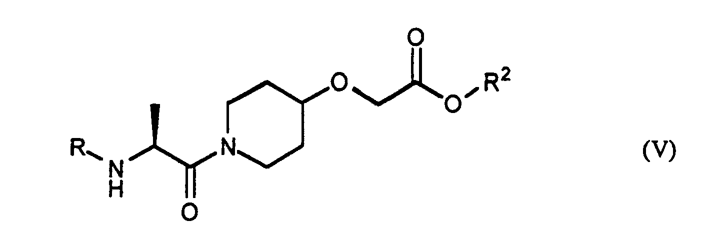 wherein R represents an amino protecting group and R² is as defined above,
b) the amino protecting group R is cleaved off,
c) if desired, the R² group is trans-esterified to the ethyl ester, with reaction steps b) and c) optionally being carried out simultaneously, and subsequently
d) the resulting amine of formula (VI) 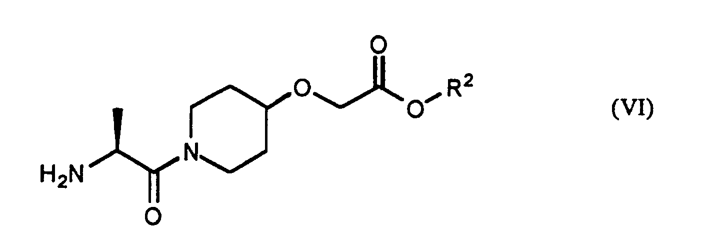 wherein R¹ is as defined above,
is converted with 4-cyanobenzoyl chloride into the compound of formula (VII) 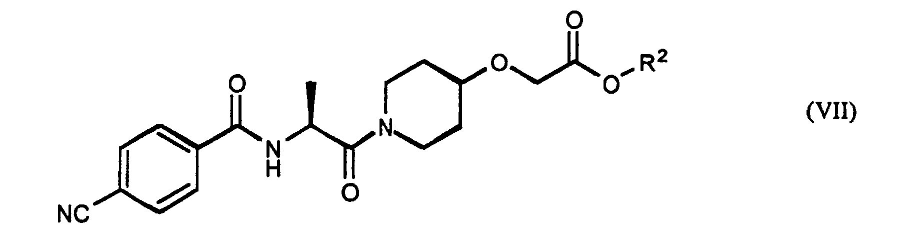 wherein the group R² is as defined above,
and subsequently, if desired, R² is trans-esterified to the ethyl ester, subsequently
e) ethyl [Z]-(S)-[[1-[2-[[4-(amino-hydroximino-methyl)-benzoyl]amino]-1-oxopropyl]-4-piperidinyl]oxy]acetate is obtained by reaction of hydroxylamine hydrochloride with the compound of formula (VII) and
f) the ethyl [Z]-(S)-[[1-[2-[[4-(amino-hydroximino-methyl)-benzoyl]amino]-1-oxopropyl]-4-piperidinyl]oxy]acetate obtained is, if desired, converted into a pharmaceutically suitable salt.

10. The use of a process according to any one of claims 1 to 9 for the manufacture of N-t-butyl-decahydro-2-[2(R)-hydroxy-4-phenyl-3(S)-[[N-(2-quinolylcarbonyl)-L-asparaginyl]-amino]butyl]-(4aS,8aS)-isoquinoline-3(S)-carboxamide or ethyl [Z]-(S)-[[1-[2-[[4-(aminohydroximino-methyl)-benzoyl]amino]-1-oxopropyl]-4-piperidinyl]oxylacetate or salts or esters derived therefrom.

## Revendications

1. Procédé pour la préparation d'anhydrides mixtes, **caractérisés en ce qu'**on ajoute une base auxiliaire au mélange d'acide et de dérivé d'acide réactif, dans lequel l'acide est un acide carboxylique, la base auxiliaire est une amine tertiaire et le dérivé d'acide réactif est un halogénure d'un acide R¹COOH ou un chloroformiate d'alkyle, R¹ étant un groupe alkyle, cycloalkyle, hétéroaryle ou aryle.

2. Procédé selon la revendication 1, **caractérisé en ce qu'**on utilise le dérivé d'acide réactif chlorure de pivaloyle, chlorure d'isovaléroyle ou chloroformiate d'éthyle.

3. Procédé selon la revendication 1, **caractérisé en ce qu'**on utilise en tant qu'acide un acide alcanecarboxylique ou un acide aminocarboxylique protégé.

4. Procédé selon la revendication 1 ou 2, **caractérisé en ce qu'**on fait réagir l'acide quinoléine-2-carboxylique avec un dérivé réactif de l'acide R¹-COOH, pour aboutir à l'anhydride quinoléine-2-carboxylique de formule (II) 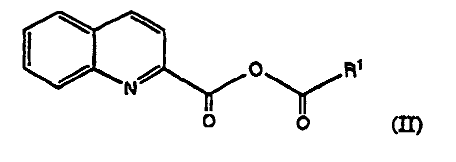 dans laquelle R¹ est un groupe alkyle, cycloalkyle, hétéroaryle ou aryle.

5. Procédé selon la revendication 4, **caractérisé en ce qu'**on fait réagir l'anhydride quinoiéine-2-carboxylique de formule (II) obtenu avec l'asparagine ou la quinargine.

6. Procédé selon la revendication 5, **caractérisé en ce qu'**on fait réagir l'anhydride quinoléine-2-carboxylique de formule (II) avec de la L-asparagine, pour aboutir à la N-(2-quinolylcarbonyl)-L-asparagine et ensuite avec le 2-[3(S)-amino-2(R)-hydroxy-4-phénylbutyl]-N-tert-butyl-décahydro-(4aS,8aS)-isoquinoléine-3(S)-carboxamide et éventuellement on convertit le N-tert-butyldécahydro-2-[2(R)-hydroxy-4-phényl-3(S)-[[N-(2-quinolylcarbonyl)-L-asparaginyl]amino]butyl]-(4aS,8aS)-isoquinoléine-3(S)-carboxamide en un sel pharmaceutiquement approprié ou en un ester pharmaceutiquement approprié.

7. Procédé selon l'une quelconque des revendications 1 à 3, **caractérisé en ce qu'**on fait réagir un acide de formule (I) 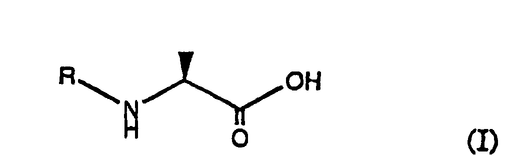 dans laquelle R représente un groupe protecteur de fonction amino, avec un dérivé réactif d'un acide R¹-COOH, pour aboutir à l'anhydride de formule (III) 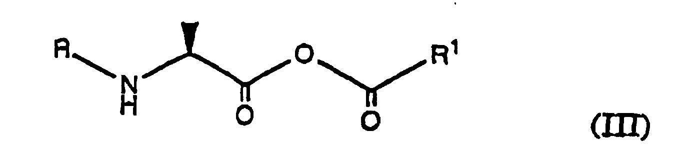 R ayant la signification donnée plus haut et R¹ étant un groupe alkyle, cycloalkyle, hétéroaryle ou aryle.

8. Procédé selon la revendication 7, pour la préparation de l'anhydride (S)-2-benzyloxycarbonylaminopropionyl-2,2-diméthylpropionique ou de l'anhydride (S)-2-tert-butoxycarbonylaminopropionyl-2,2-diméthylpropionique par la réaction de l'acide (S)-2-benzyloxycarbonylaminopropionique ou de l'acide (S)-2-tert-butyloxycarbonylaminopropionique avec un dérivé d'acide réactif de l'acide 2,2-diméthylpropionique.

9. Procédé selon la revendication 7 ou 8, **caractérisé en ce qu'**on convertit l'anhydride
a) par la réaction avec un composé de formule (IV) 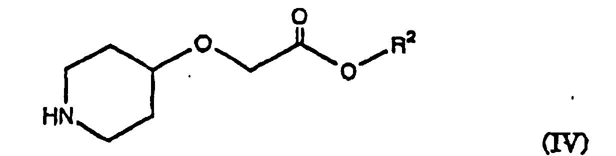 dans laquelle R² représente un groupe alkyle,
en un ester de formule (V) 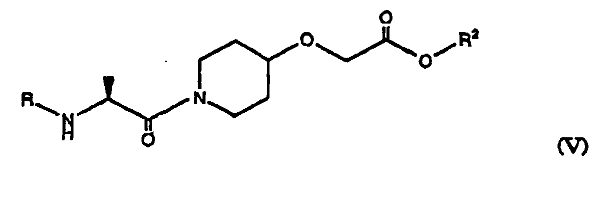 dans laquelle R représente un groupe protecteur de fonction amino, et R² est tel que défini ci-dessus,
b) on élimine le groupe protecteur R de fonction amino et
c) on transestérifie éventuellement le groupe R² en l'ester méthylique, les étapes de réaction b) et c) pouvant éventuellement avoir lieu simultanément, et ensuite
d) on fait réagir l'amine obtenue, de formule (VI) 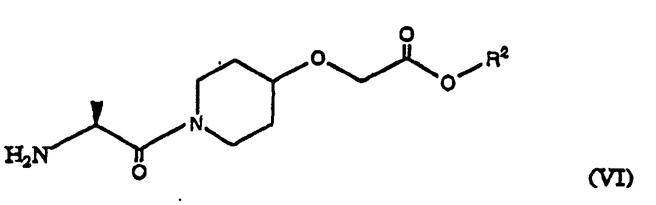 dans laquelle R² est tel que défini plus haut, avec du chlorure de 4-cyanobenzoyle , pour aboutir au composé de formule (VII) 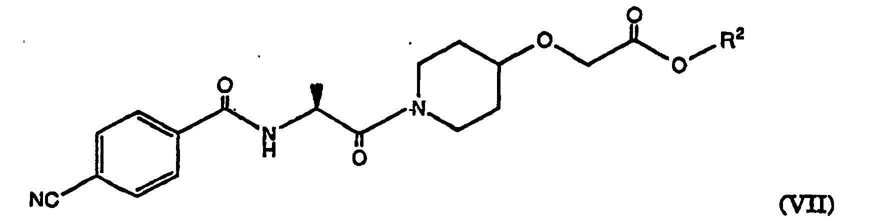 le groupe R² étant tel que défini plus haut, et ensuite R² est éventuellement transestérifié en l'ester éthylique, ensuite
e) on obtient le [Z]-(S)-[[1-[2-[[4-(aminohydroxyiminométhyl)benzoyl]amino]-1-oxopropyl]-4-pipéridinyl]oxy]acétate d'éthyle, par la réaction du chlorhydrate d'hydroxylamine avec le composé de formule (VII), et
f) le [Z]-(S)-[[1-[2-[[4-(aminohydroxyimlnométhyl)benzoyl]amino]-1-oxopropyl]-4-pipéridinyl]oxy]acétate d'éthyle est éventuellement converti en un sel pharmaceutiquement approprié.

10. Utilisation d'un procédé selon l'une quelconque des revendications 1 à 9, pour la préparation du N-tert-butyldécahydro-2-[2(R)-hydroxy-4-phényl-3(S)-[[N-(2-quinolylcarbonyl)-L-asparaginyl]amino]butyl]-(4aS,8aS)-isoquinoléine-3(S)-carboxamide ou du [Z]-(S)-[[1-[2-[[4-(aminohydroxylminométhyl)benzoyl]-amino]-1-oxopropyl]-4-pipéridinyl]oxy]acétate d'éthyle ou de sels ou d'esters dérivés de ceux-ci.
